# EUROPEAN PATENT APPLICATION

(11) **EP 4 483 934 A1**
(43) Date of publication of application: **01.01.2025**
(21) Application number: 23758931.2
(22) Date of filing: 17.01.2023
(51) Int. Cl.: A61M 25/00

(54) **MEDICAL CATHETER**

(30) Priority: 22.02.2022 CN 202210163396
(71) Applicant: Shangai Kegang Medical Technology Co., Ltd, Shanghai 201613 (CN)
(72) Inventor: WANG, Wenzhe, Shanghai 200135 (CN); ZHANG, Zhichao, Shanghai 200135 (CN); RU, Chengtao, Shanghai 200135 (CN); ZHAO, Chenlie, Shanghai 200135 (CN); MA, Changsheng, Shanghai 200135 (CN); DONG, Jianzeng, Shanghai 200135 (CN)
(74) Representative: Cameron Intellectual Property Ltd
(86) International application number: PCT/CN2023/072522
(87) International publication number: WO 2023/160306

(57) **Abstract**

A medical catheter (1) comprising a proximal end and a distal end. The distal end comprises a sensor array (32) used for displaying a lumen inner wall (51); a coaxial rotation device, with a through hole in communication with the proximal end of the catheter (1). The through hole is for establishing a channel between a target bifurcated lumen (52) and the catheter distal end (1). A lumen internal fixing device (13) is provided for fixing relative positions of the catheter distal end (1) and the lumen inner wall (51), so as to ensure the stability of establishing a channel between the bifurcated lumen (52) and the catheter proximal end (1). The medical catheter detects the structure and distribution of the lumen (51) in a human body, and can conveniently guide an operator to probe into the target bifurcated lumen (52) to satisfy the requirements of a surgical operation.

## Description

### TECHNICAL FIELD

The invention relates to the field of medical device, in particular to a medical catheter.

### BACKGROUND

The main navigation method in interventional surgery is to rely on DSA (Digital Silhouette Technology) to use X-rays to develop the device during the operation, so as to assist the operator to determine the relative position or relationship between the interventional device and the patient's body. Surrounding tissue development, comparison of pre-operative images (CT, MRI, DSA, etc.) and operator experience are used to determine the specific location of the device.

Due to the extensive use of DSA, doctors and patients will be exposed to ionizing radiation for a long time, which has hidden damage to the health of doctors and patients. Therefore, in recent years, more auxiliary imaging techniques have been used in interventional surgery. Such techniques include:
1. Electromagnetic navigation, using the frequency matching of the electromagnetic coil on the catheter and the frequency of the external electromagnetic coil to determine the position of the catheter in the body. However, this method can only determine or know the spatial position of the device, while it cannot directly image the patient's tissue. This technique is now commonly used for intra-atrial navigation. The tactile sensor at the tip of the catheter is used together with the electromagnetic coil to record the absolute position of the space, while the tip of the catheter is in contact with the atrial wall. By touching more points in the atrium, the atrium contour is outlined to create a tissue image. Since the inner wall of the atrium is shaped like a convex envelope, a more accurate inner wall of the atrium can be roughly outlined by using the absolute positions of multiple points in the atrium by using this approximation algorithm. However, this imaging method cannot accurately outline the rugged inner wall, especially the multi-branched human internal lumen.
2. Intracardiac Echocardiographic, using ultrasound to show the slices of the heart. This method can precisely visualize the contour and location of tissues and/or devices with respect to different acoustic characteristics. However, the images obtained in this way are the cross-sections of several two-dimensional images, which requires the operator to conceive or visualize the spatial state proficiently to determine the relative position of the device and the tissue. At the same time, the placement position and scanning range of the ultrasonic catheter also need to be adjusted by the operator, which is inconvenient. Due to the need for clear imaging of the entire cardiac cavity, the ultrasonic array has a great number of sensors and a large volume, which cannot be used in narrow and rugged lumen.
3. Compared to Intracardiac Echocardiographic, intracavity ultrasound pays more attention to the tissue structure distribution of the lumen wall, and it is used to guide the surgeon to treat the cavity in the target area. This has led to its attribute of focusing on the detection of the epidermis in the cavity. At the same time, the relative relationship between the catheter and the patient still needs image assistance such as DSA. For the detecting branch cavity, the doctor still needs to use experience and operation to explore the lumen.

On the other hand, DSA can only provide projections in one direction at the same time, and it cannot have a complete presentation of the structure of the spatially distributed branches, making it more difficult to probe into the spatially distributed branch.

Catheters used in the arterial system in medicine now use radiopaque materials or spray radiopaque materials in conjunction with digital silhouette technology (DSA) for catheter positioning. Because of the coarse-to-thin transfer property of arterial blood, the target site can be visualized by spraying the aorta or branch arteries near the target site with a radiopaque liquid, such as iohexol. But for the venous system, because the blood flows back to the heart, it is impossible to develop the thinner blood vessels by spraying the contrast agent on the venous trunk of the blood supply system. It is often easy for doctors to enter the venous system to enter the main trunk of the venous system, but it is difficult to enter the branches of the venous system, especially the fine branches.

Therefore, there is an urgent need to provide a new catheter design for detecting the structure and distribution of the human body cavity, and at the same time, it can conveniently guide the operator into the target bifurcation cavity to meet the needs of subsequent operations.

### SUMMARY

The purpose of the present invention is to solve the problem that existing medical catheters have difficulty entering the branches of the venous system, especially the distal branches. The application provides a new medical catheter design, which is used to detect the structure and distribution of the human body's internal cavity, and can simultaneously make it convenient to guide the surgeon into the target bifurcation cavity to meet the needs of subsequent operations.

The present invention provides a medical catheter, comprising a proximal end and a distal end. The distal end comprises, a sensor array to show the condition of the tube or inner wall in the lumen. A coaxial rotating device, which can rotate independently relative to other parts of the catheter, has a through hole connected to and in communication with the outside of the catheter, and is used to establish the through hole to form the channel between the target branch lumen and the inside of the catheter.

A fixing device is used to fix the relative position of the distal end of the catheter and the inner wall of the lumen, to ensure the stability of the process of establishing the channel between the target branch lumen and the inside or inner side of the catheter.

The sensor array can guide a distal hole of the rotating part to align the through hole with the target lumen. Each individual sensor unit in the sensor array can send and receive signals independently, that is, the signal properties and emission time can be independently controlled. The sensor array can guide the holes of the coaxial rotating device to align with the target lumen by fully or entirely displaying or partially displaying the contour of the inner wall. The detection mode of the sensor array can be uniformly distributed at the distal end of the catheter, or can be distributed in any fan-shaped area or radial arrangement around or substantially surrounding the rotating or rotatable part of the catheter. The sensor array is an array of ultrasonic transducers for transmitting and receiving ultrasonic signals for detecting the inner wall contour of the lumen or cavity.

Ultrasonic transducer arrays. Adjacent ultrasonic transducer arrays need to transmit and receive signals successively, and the arrays on both sides can simultaneously transmit and receive signals to improve imaging efficiency. An ultrasonic transducer array capable of resolving or identifying an opening or hole with a diameter of 0.3 mm is sufficient to meet the desired requirements.

The sensor array can also be an electromagnetic sensor or a photoelectric sensor, which is used to directly feedback the position information of the distal end of the rotatable part of the device relative to the main body of the catheter. The sensor array can also be an optical sensor, such as optical interference imaging, a miniature wide-angle lens or a laser transceiver.

The fixation or fixing device can be a balloon or an elastic support. The fixing device can also be a Nitinol stent or a multi-link mechanism composed of a hard material with good biocompatibility (titanium alloy or stainless steel e.g.). The fixing device has a driving end at the proximal end of the catheter, and the fixing device can be manually or automatically driven to switch states or generate deformation, so that it can stabilize the relative position between the distal end of the catheter and the lumen.

The rotation mode of the coaxial rotating device or coaxial rotating part of the device, can be manually moved or driven by a computer-controlled motor. The coaxial rotating device can rotate independently relative to other parts of the catheter, and its rotation angle can be controlled. The proximal end of the catheter and the coaxial rotating device are connected to a firm or rigid mechanical structure. The driving torque of the coaxial rotating device can be connected at the proximal end of the catheter through a flexible shaft that can transmit torque, and feedback about the distal end of the coaxial rotating device is provided by controlling the proximal end of the coaxial rotating device, such as by provided micro motors. The proximal end and the distal end of the coaxial rotating device are connected by a flexible shaft that can rotate together or synchronously. Through the directional rotation of the coaxial rotating device, the hole on the coaxial rotating device can be rotated so that it is angularly aligned with the target branch lumen, which helps to establish the positional relationship of the channel along and between the hole and the target branch lumen. The relative angular relationship between the coaxial rotating device and the catheter can be provided by the structure at the proximal end and/or the set of position sensors at the distal end. The relative angular relationship between the coaxial rotating device and the catheter can transmit data in real time to indicate position to the operator and/or an automatic control system. The coaxial rotary device can guide the microcatheter or guide wire provided via a proximal inlet from the proximal end of the catheter, through the lumen or through hole of the catheter and out of the distal hole on the coaxial rotary device at the distal end of the catheter. Alternatively, the coaxial rotary device can directionally release devices or prefilled substances at the distal end of the catheter, such as coils, lumen plugs, or drugs, to be delivered to the distal branch lumen.

Preferably, the distal end of the catheter may contain a distal positioning device. The distal end of the catheter may contain a radiopaque material, or the radiopaque material may be sprayed to achieve the effect of imaging under radiation; or a coil or set of orthogonally placed coils to achieve positioning in a magnetic field, or a series of electrodes that transmit electrical signals to achieve positioning in an electric field. The previously mentioned design can be realized by the operator and/or the automatic control system for judging the relative position of the distal end of the catheter relative to the human body and/or adjacent organs.

The device of this application can be summarized as follows:
a) An array of ultrasonic transducers are uniformly distributed, preferably in a radial pattern or arrangement, at or around the distal end of the catheter. In any sector, with the catheter as the origin, there is a group of ultrasonic transducer arrays for transmitting and receiving ultrasonic signals for detecting the inner wall contour of the cavity. In this arrangement, adjacent ultrasonic transducer arrays need to transmit and receive signals successively, and the arrays on both sides, or on opposing sides of the catheter, can simultaneously transmit and receive signals to improve imaging efficiency. On the other hand, due to the limitation of catheter diameter and ultrasonic transducer size, the number of ultrasonic transducers in the array will be reduced, but as the area of primary concern is the structure and condition of the lumen or cavity wall, the resolution is sufficient to resolve bifurcations with a diameter of 0.3 mm to meet the necessary identification requirements. In this device, the coaxial rotatable device is coaxially rotatable with the catheter body, and its rotational position relative to the catheter body needs to be controllable, such as by a reliable mechanical connection between the catheter and the proximal end of the coaxially rotatable device. The proximal end and the distal end of the coaxial rotatable device are preferably connected with a flexible shaft and rotate together or synchronously. Feedback about the distal end of the rotatable device is provided by control of the proximal end of the coaxial rotatable device, especially at the distal end to establish the position and relationship of holes and target branch lumens to form the channel. Another example is that using an electromagnetic sensor or a photoelectric sensor installed at the distal end of the catheter, direct feedback is provided about the position information of the distal end of the rotatable device relative to the main body of the catheter.
b) Using a group of ultrasonic transducer arrays or optical sensors such as near-infrared optical interference imaging (OCT), miniature wide-angle lenses or other optical interference imaging devices directly connected to the coaxial rotatable device, the sector range of the array scanning can be simultaneously provided at a distal port of the shaft rotation device. At any moment when the catheter enters the cavity, the coaxial rotatable device rotates at high speed and rotates to any sector at the same time, the ultrasonic array or optical sensor uses phase control technology or interference scanning to quickly scan the acoustic signals of each point in the sector and provides real-time feedback. After the computer receives the signal, the images are combined to provide rebuild-information on the structure of the inner wall of the surgeon's cavity of interest. Under this arrangement, the efficiency of converting the ultrasonic transducer array or optical sensor into an image is high, so the position of the cavity wall can be estimated by using the shape of the fixture deployed in the cavity. Therefore, an algorithm can be optimized so that the scanning range of the ultrasonic transducer or optical sensor is focused on the vicinity of the cavity wall, thereby reducing the amount of signal sending and receiving and associated calculations. When the operator finds the target branch of the lumen, the posture or position can be finely manipulated with the coaxially rotatable device to see the branch of the lumen and stop, and further control the alignment of the distal hole opening with the target branch lumen.

The intraluminal fixation device can be a balloon located at the distal end of the catheter that does not affect channel establishment, a memory metal stent such as a nickel-titanium stent, or a multi-link mechanism composed of a hard material with good biocompatibility (titanium alloy or stainless steel). Its driving end is used to deploy the fixation device at the proximal end of the catheter to ensure that the relative position of the distal end of the catheter and the target lumen is fixed.

The distal positioning device of the catheter can be made of a radiopaque material or a similar effect can be achieved by spraying a radiopaque material according to the usage scenario and requirements. It can be a set of orthogonally placed coils, or a series of coils that can transmit electrical signals the electrodes. In some scenarios, such as different flow velocities inside the lumen or differences in the basic properties of the substances in the lumen, such as pH value or chemical composition, the specific position of the distal end of the catheter in the lumen can be known by using a matching sensor.

The beneficial effect of this invention is that it can greatly reduce the difficulty and complexity of the surgical operation, reduce the use time of DSA, increase the success rate of accurate device placement and operation success rate, thereby benefiting patients and doctors. The invention provides a technical solution for detecting the contour of the inner wall of the human body's internal cavity and precisely guiding it into the subdivided branches, including structural design, sensor arrangement, and control and algorithm of supporting catheters. The invention includes ultrasonic and optical sensors, related imaging and navigation algorithms and a guiding and positioning structure, which are used for real-time detection of the inner wall contour of the human body's internal cavity, and it can assist in guiding into the target side cavity. The invention provides a technical means for doctors to conveniently send a guidewire or catheter into the tiny bifurcations of the patient's venous system. At present, the success rate of doctors operating a guide wire into the target branch vein under the guidance of DSA is 40%-95%, and only a few doctors can achieve a success rate of more than 90%. Using the present invention, the success rate of accessing branch veins can reach nearly 100%.

On the other hand, compared to the situation where some doctors use existing intraluminal imaging catheters in combination with guide wire catheters to solve similar problems, the present invention does not require the doctor to repeatedly insert and pull out the intracavity imaging catheter to provide space for the guide wire catheter. Through the optimization of the algorithm, the demand for product hardware is reduced, and the cost is further reduced compared with the existing intracavity imaging catheter.

In order to make the above-mentioned objects, features and advantages of the present invention more comprehensible, preferred embodiments will be described in detail below together with the accompanying drawings.

### DESCRIPTION OF THE DRAWINGS

In order to more clearly illustrate the specific implementation of the present invention or the technical solutions in the prior art, the following will briefly introduce the accompanying drawings that need to be used in the specific implementation or description of the prior art. Obviously, the accompanying drawings in the following description show some implementations of the present invention, and those skilled in the art can obtain other devices based on these drawings without any creative effort.
Figure 1 is an overall schematic diagram of the product according to one embodiment of the present invention.
Figure 2 is an overall schematic diagram of the catheter part according to one embodiment of the present invention.
Figure 3 is a detailed schematic view of the catheter connector according to one embodiment of the present invention.
Figure 4 is a schematic diagram of the delivery state according to an embodiment of the present invention.
Figure 5 is a schematic diagram of an expanded state of an embodiment of the present invention.
Figure 6 is a schematic diagram of ultrasonic detection according to an embodiment of the present invention.
Figures 7a, 7b, and 7c are ultrasound display images according to an embodiment of the present invention.
Figure 8 is a schematic diagram of the rotation part of an embodiment of the present invention.
Fig. 9 is a schematic diagram of a rotary encoder according to another embodiment of the present invention.
Fig. 10 is a cross-sectional view of an embodiment of the invention in rotation and showing the through hole.
Fig. 11 is a schematic diagram of a rotating part with a sensor according to another embodiment of the present invention.
12a and 12b are schematic diagrams of the process of detecting the inner wall of a cavity according to an embodiment of the present invention.
Fig. 13 is a schematic diagram of a balloon embodiment of an embodiment of the present invention.
Fig. 14 is a schematic diagram of a practical operation demonstration of an embodiment of the present invention.
Fig. 15 is a schematic diagram of a second practical operation demonstration of an embodiment of the present invention.

### DETAILED DESCRIPTION

The technical solutions in the embodiments of the present invention will be clearly and completely described below in conjunction with the accompanying drawings of the embodiments of the present invention. Obviously, the described embodiments are only some of the embodiments of the present invention, but not all of the embodiments. The components of the embodiments of the invention generally described and illustrated in the figures herein may be arranged and designed in a variety of different configurations. Therefore, the following detailed description of the embodiments of the invention provided in the appended drawings is not intended to limit the scope of the claimed invention, but rather to represent selected embodiments of the invention. Based on the embodiments of the present invention, all other embodiments obtained by those skilled in the art without any creative work fall within the scope of protection of the present invention.

The present invention will be described in further detail below, so that those skilled in the art can implement it with reference to the explanatory text.

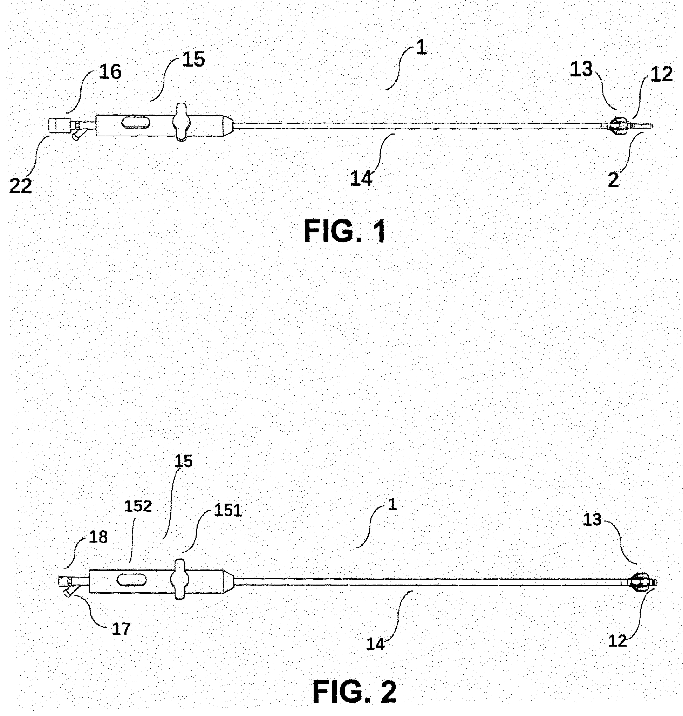

First please refer to FIG. 1, which is an overall schematic diagram of a product according to an embodiment of the present invention. This embodiment is a specific implementation of an array of ultrasonic transducers uniformly distributed radially at the distal end of the catheter. In Figure 1, it includes a catheter 1, a rotating part 2, an ultrasonic array 12, an intraluminal fixation device 13, a steerable catheter 14, a handle 15, a catheter connector and a stator connector 16, and a rotor part 22.

Please refer to FIG. 2 next. FIG. 2 is an overall schematic diagram of a catheter part according to an embodiment of the present invention. The catheter 1 of the present invention includes a catheter fixing connector 18, a perfusion port 17, a handle 15, a steerable catheter 14, an intraluminal fixation module 13 and an ultrasound array 12 from the proximal end to the distal end. The perfusion port 17 is used to inject physiological saline mixed with low molecular weight heparin or other anticoagulants suitable for the patient in advance to ensure that no emboli are generated, or as little emboli are generated as possible, when the catheter 1 is inserted into the patient's body. The handle 15 includes a knob 151 and a sliding button 152. The knob 151 drives two high-strength wires embedded in the steering catheter 14 to drive the bending of the steering catheter 14. The sliding button 152 drives the slide 132 forward and backward along a central axis of the catheter, and moves to drive the opening and closing of the intraluminal fixation module 13 .

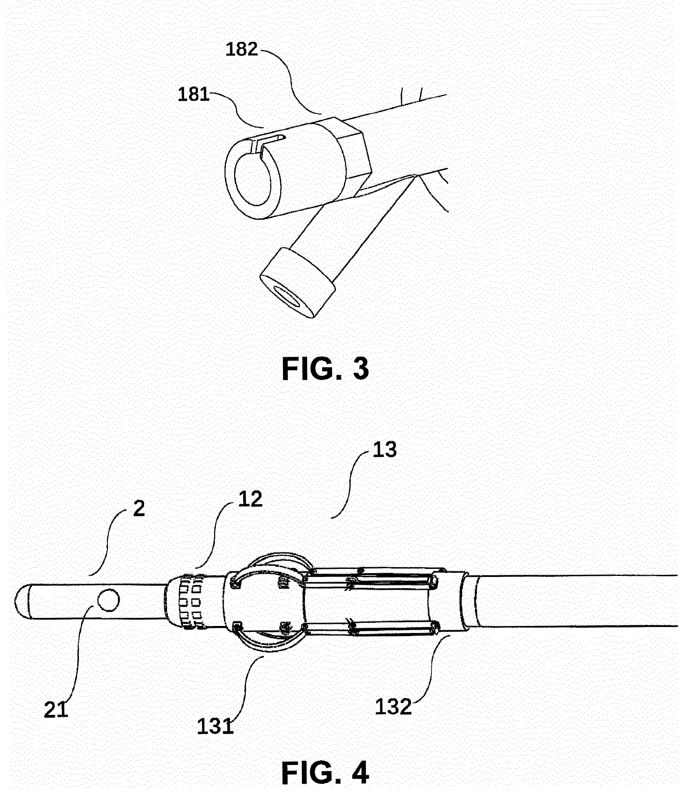

FIG. 3 is a detailed diagram of a catheter fixing connector according to an embodiment of the present invention. The conduit fixing connector 18 is mainly used to fix the rotating part 2. There is a characteristic port 181 on the connector, which is matched with the characteristic port of the stator part 23 of the rotating part 2, and is used to provide the only reliable connection direction, aiming to ensure the connection and direction is certain. After the rotating part 2 is connected with the stator part 23, rotating the fixing knob 182 can ensure the stable connection between the rotating part 2 and the stator part 23 .

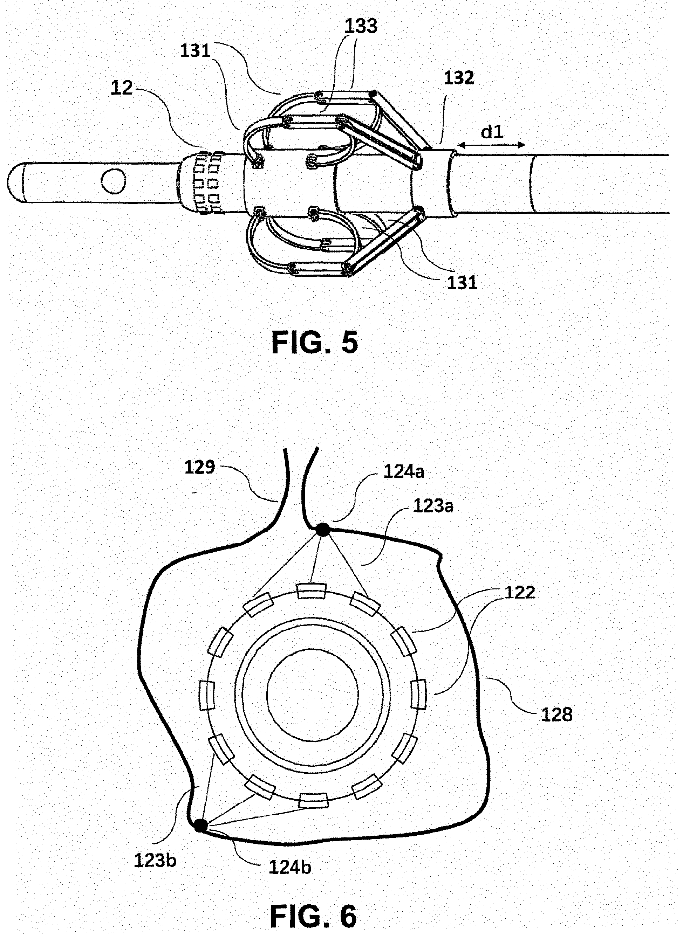

Please refer to FIG. 4 and FIG. 5. FIG. 4 is a schematic diagram of a delivery state according to an embodiment of the present invention. When the slide 132 is closest to the proximal end of the catheter 1, the intraluminal fixation module 13 is in a constricted or compact state, and each part of the intraluminal fixation module 13 is in a state of close contact with the outer wall of the catheter 1, ensuring the passability of the catheter 1. The elastic bracket 131 of the intraluminal fixation module 13 is made of super-elastic metal material, which can be further tightened when being squeezed from the outside to further improve the passability. As the slide 132 slides toward the distal end of the catheter 1, the intraluminal fixation module 13 is unfolded or expanded, and the support bracket 133 is moved close to the lumen wall. The support bracket 133 can be made of radiopaque material or have coils embedded inside to meet the desired requirements. For the detection of electromagnetic signals, the elastic support 131 can ensure that the elastic force around the catheter 1 remains consistent to ensure that the axis of the catheter is as consistent as possible with the axis of the lumen. In addition, the push distance d1 of the slide 132 is different from that of the external drive.

The moving distance of the sliding button 152 is consistent, and when the stress of the elastic bracket 131 is greater than a certain value, the elastic bracket 131 will drive the slide 132 to retract until the stress on the elastic bracket 131 reaches a reasonable range. In this way, the specific position of the support bracket 133 can be obtained by the specific position of the external driving slide button 152, thereby further obtaining the approximate diameter of the inner wall of the current lumen.

FIG. 6 is a schematic diagram illustrating the use of ultrasonic detection according to an embodiment of the present invention. FIG. 6 shows the specific implementation of the extra-catheter ultrasound array 12 in the lumen. The distal end of the catheter 1 includes an extra-catheter ultrasonic array module 12, wherein the ultrasonic transducer group 122 is evenly distributed on the outer wall of the lumen along the circumferential direction. Each of its ultrasonic transducers can send and receive signals independently. The ultrasonic transducer group 122 emits an ultrasonic signal group 123a and another ultrasonic signal group 123b at the same time for detecting two points 124a and 124b in the space. After the two groups of ultrasonic arrays, respectively, scan the area, they move clockwise or counterclockwise, and rotate the position of an ultrasound transducer to re-scan the new area to construct the ultrasound image of the section.

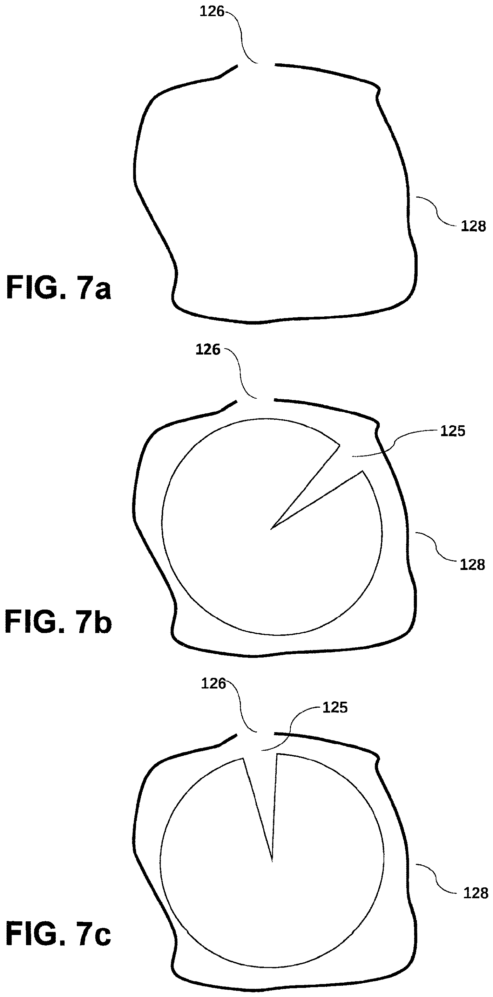

Figures 7a, 7b, and 7c are ultrasound display images according to an embodiment of the present invention. In this embodiment, the ultrasound catheter detects the lumen 128 and the bifurcation 129, and the image in Figure 7a can be obtained. When used specifically, the catheter 1 enters the target lumen through the previously preset sheath channel in a bundled, constricted or collapsed state, and in vitro imaging is used to detect the intraluminal fixation device 13 at the distal end of the catheter 1, and to determine its position in the approximate area of interest. Once located, and driving the sliding button 152 opens or expands the fixation device 13 in the cavity so that it is in close contact with the inner wall of the cavity. The ultrasonic transducer group 122 of the catheter 1 is used to find the bifurcation of interest in the lumen. When the ultrasonic transducer group 122 successfully finds the bifurcation that may be of interest, it starts to drive the rotor part of rotating part 2 of the catheter.

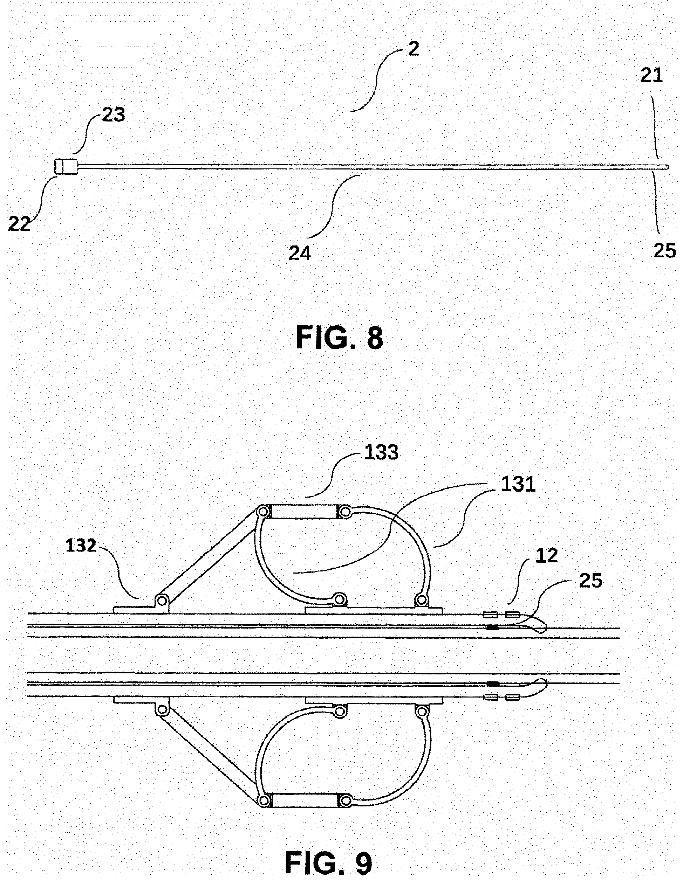

FIG.8 is a schematic diagram of the rotation part according to an embodiment of the present invention. The proximal end of the rotating part 2 contains a set of rotor parts 22 and stator parts 23 that have been assembled. The stator part 23 and the fixed connector 18 of the catheter 1 are locked and connected to each other and cannot rotate with respect to each other. The rotor part 22 connects to the torque transmission tube 24 of the rotating part 2 and cannot rotate with respect to each other. The torque transmission tube 24 can be a braided tube or may also be a hollow flexible shaft, aiming to reliably transmit the rotation of the proximal rotor part 22 to the rotation and position of the distal side hole 21. The rotor part 22 rotates coaxially with the stator part 23 and the angle between the two may be indicated. In the present invention, one implementation is that the origin of the stator part 23 is aligned with a certain group of ultrasonic transducer groups 122 at the distal end of the catheter 1. The hole 21 is aligned when the image prompts that the angle between the corresponding position of the bifurcation opening or notch and the origin of the ultrasonic transducer group 122 is α degrees. The alignment can thus be achieved by rotating the rotor part 22 so that the origin of the rotor part 22 is aligned with the α degree position to control the distal rotor.

FIG. 9 is a schematic diagram showing a rotary encoder 25 according to another embodiment of the present invention. In this embodiment, directly reading the position of the distal end of the rotating part can control the distal side hole 21 more reliably. After the distal end of the rotating part 2 is matched with the distal end of the catheter 1, the position of rotating part 2 relative to catheter 1 is read by the encoder 25. A fixed ultrasonic transducer inside the catheter 1 is defined as the origin, and a signal emission source, such as an optical signal emission source, is placed on the corresponding inner wall of the catheter. After the encoder 25 receives the optical signal, it can obtain the specific angle of the currently rotating part 2. The rotor part 22 and the stator part 23 can also be the rotor and the stator of a servo motor or a stepping motor, which are controlled by external signals to rotate precisely. See 7(b) and 7(c) in Figure 7 showing the specific embodiment. The distal side hole 21 of the rotating part 2 will appear as a notch 125 on the image. By rotating the rotating part 2, the notch 125 is aligned with the detected bifurcation opening 126, that is, the through hole or standard distal side hole 21, is aligned with the bifurcation opening 126 of interest.

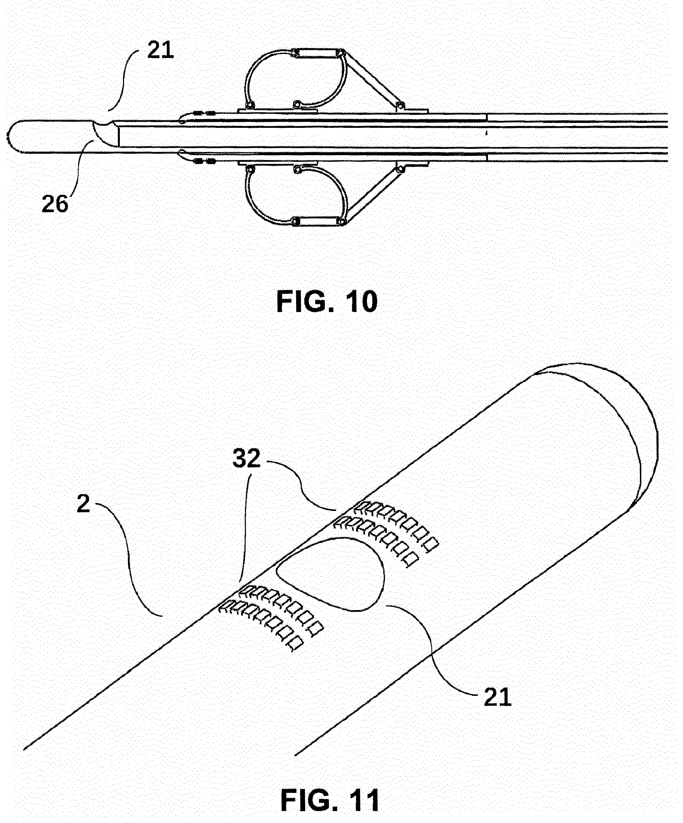

FIG. 10 shows that by manipulating the rotating part 2, the distal side hole 21 of the rotating part 2 can be precisely aligned with the target lumen. The distal side hole 21 of the rotating part 2 is connected to the proximal connector of the rotating part 2, so that the operator can insert the microcatheter or the guide wire. The inner structure of the distal side hole of the rotating part 2 has a guide surface 26 for guiding the guide wire or microcatheter to protrude from the distal side hole.

FIG. 11 shows a schematic diagram of the rotating part with a sensor according to another embodiment of the present invention. The rotating part 2 with sensors is placed with the sensor arrays 32 on the rotating part, and a sensor array 32 is distributed on both sides of the distal side hole 21 along the central axial direction of the catheter, and the radial distribution only covers or extends along width of the distal side hole 21. The sensor array 32 may be a set of ultrasonic arrays, or an infrared optical sensor for detecting the contour of the surrounding inner wall using the rotating part 2 with sensors.

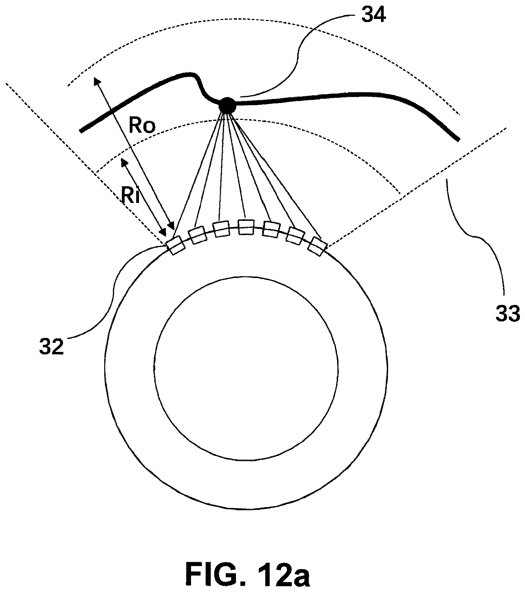

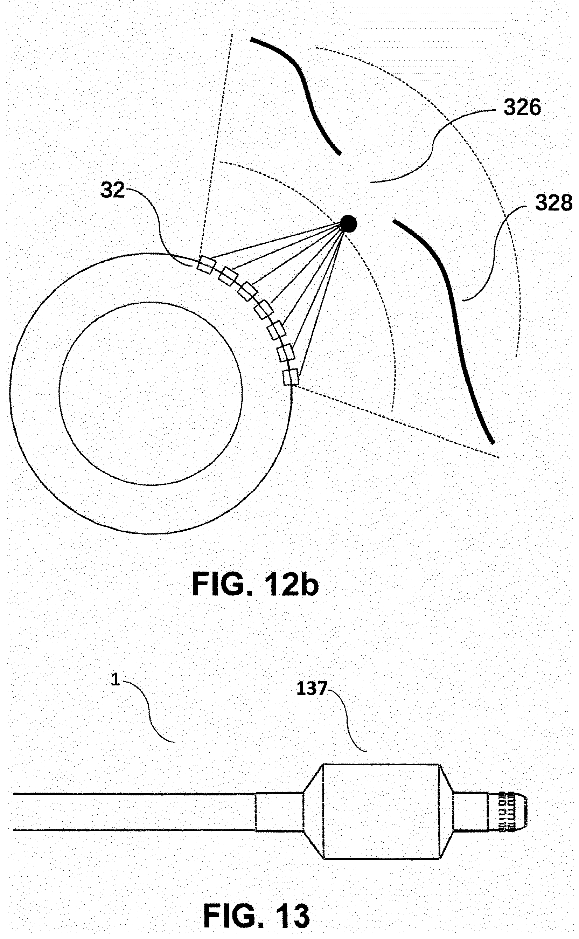

Please refer to Figures 12a and 12b. Figures 12a and 12b are schematic diagrams of the process of detecting the inner wall of a cavity according to an embodiment of the present invention. As shown in Figures 12a and 12b, in this embodiment, the stator 23 is still reliably connected to the fixed connector 18 of the catheter, and the rotor 22 is driven to rotate by the motor, thereby driving the entire rotating part 2 to rotate, while the sensor 32 performs high-speed sampling of the surrounding inner wall of the cavity. Figures 12a and 12b show the process of detecting the inner wall of the cavity. Each signal generator on the sensor array 32 can independently control the emission, by constructing a detection focal point 34 for the time difference of emission. Since the image is acquired by the sensor array 32 in real time, while the sensor array 32 is rotating, a higher image acquisition efficiency is required. The specific position of a support bracket 133 can be obtained through the specific position of the slide button 152 on the catheter 1, so that the approximate diameter of the inner wall of the lumen can be further obtained. Additionally, the diameter of the inscribed circle of the inscribed square of the estimated approximate diameter can be used as the range of interest from the minimum value Ri to the circumscribed circle of the circumscribed square of the estimated diameter of the maximum value Ro, so that the range of interest can greatly reduce the points of sweeping or scanning, thereby reducing the amount of calculation in the area 33 surrounded by the dashed line in Figures 12a and 12b. That is the area that is scanned each time.

Likewise, the arrival of the catheter 1 and the rotating part 2 at the area of interest can be estimated by the position of the intraluminal fixation module 13 relative to the patient. Looking for bifurcations of interest by rotating part 2 of the probe, Figures 12a and 12b show the case where a fork of interest is detected in the lumen 328. When the sensor array 32 detects the bifurcation 326, the motor controls the rotor 22 to be fixed at the position facing the bifurcation 326. In this illustrated embodiment, the sensor array 32 is close to the distal side hole 21 of the rotating part 2. Therefore, it can be ensured that the distal side hole 21 of the rotating part 2 is aligned with the bifurcation 326 . Similarly, the operator can guide the guide wire or microcatheter into the target bifurcation of interest through the connection or channel between the proximal inlet to the through hole and the distal side hole 21.

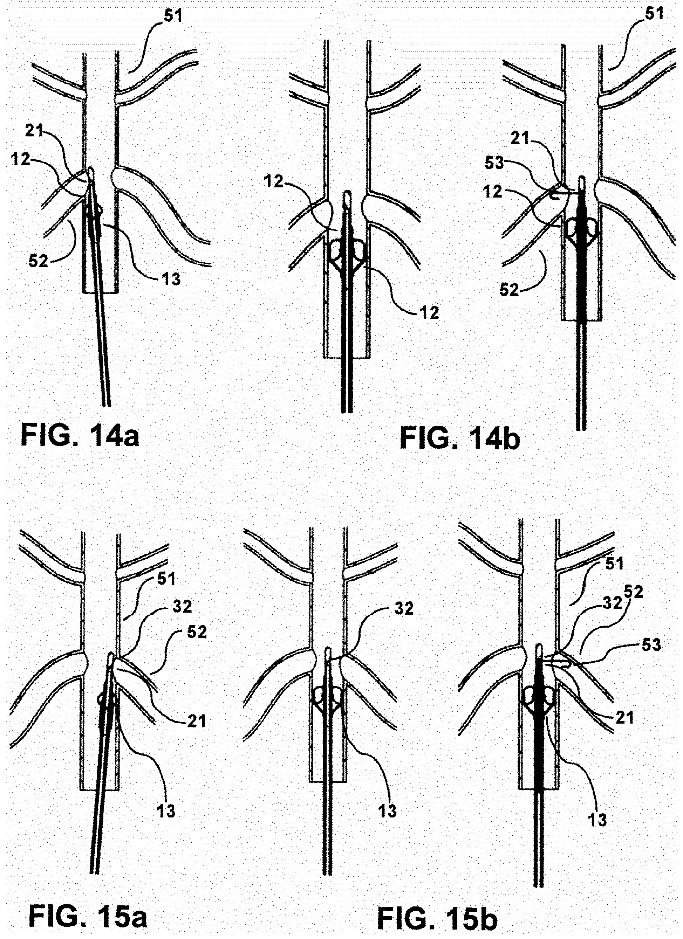

FIG. 13 is a schematic diagram of a balloon embodiment of an embodiment of the present invention. As shown in FIG. 13, another embodiment of the intraluminal fixation device 13 of the embodiment is to use a compliant balloon 137 to perform intraluminal fixation of the catheter 1 by filling and discharging the balloon 137 . Furthermore, the diameter of the lumen where the catheter is located can also be estimated by changing the amount of liquid injected and the pressure within the balloon 137 . In some practices, a direct assessment of the lumen diameter can be achieved by inflating balloon 137 with a radiopaque liquid. FIG. 14 is a practical surgical operation demonstration 1 of an embodiment of the present invention.

As shown in Figure 14(a) the catheter enters the lumen 51 in a constricted state. At this time, the detection of the ultrasonic array 12 on the catheter forms a large angle with the axis of the lumen, and a stable image cannot be obtained when moving or shaking the lumen. Through the deployment of the fixation device 13 in the lumen, as shown in FIGURES 14(b), the central longitudinal axis of the catheter is basically consistent with the axis of the lumen, and the ultrasonic array 12 can detect the target lumen 52 and stabilize it near the target. The rotating part 2 is then manipulated so that its distal side hole 21 is aligned with the bifurcation of interest 52, and then the guide wire 53 is inserted into the target bifurcation of interest 52 from the distal side hole as shown.

Figure 15 is a second practical surgical operation demonstration of an embodiment of the present invention. As shown in Figure 15(a), the catheter enters the cavity 51 in a constricted state. At this time, the detection of the sensor array 32 on the rotating part forms a large angle with the axis of the cavity, and a stable image cannot be obtained. Through the deployment of the intracavity fixing device 13, as shown in FIG. 15(b), the sensor array 32 on the rotating part can scan and display the inner wall of the target lumen. When it is confirmed that the bifurcated lumen seen is the target bifurcated lumen 52, the motor is manipulated so that the sensor array 32 is locked and focused on the bifurcated lumen 52, and then the guide wire 53 is passed through the distal side hole 21 directly into the target lumen fork 52 as shown.

Although embodiments of the present invention have been disclosed above, they are not limited to the uses set forth in the specification and description. It can be applied to various fields suitable for the present invention. Additional modifications may be readily implemented by those skilled in the art. Therefore, without departing from the claims and with the general concept being defined in scope, the invention is not limited to the specific details and illustrations shown and described herein.

## Claims

1. A medical catheter, including a proximal end and a distal end, the distal end of embodiment includes:
a sensor array for displaying the condition of an inner wall within a lumen;
a coaxial rotating device, which can rotate independently relative to other parts of the catheter, has a through hole connected to the outside of the catheter, and is used to establish a channel between a target branch bifurcation lumen and the lumen of the catheter;
a fixation device used to fix the relative position of the distal end of the catheter and the inner wall within the lumen to ensure a stable process of establishing a channel between the target branch bifurcation lumen and the lumen of the catheter; and
the sensor array can guide a distal hole of the coaxial rotating device with the through hole to align with the target branch bifurcation lumen.

2. A medical catheter as claimed in claim 1, wherein each individual sensor unit in the sensor array can independently send and receive signals, that is, signal properties and emission time can be independently controlled.

3. A medical catheter as claimed in claim 1, wherein the sensor array can guide the through hole of the coaxial rotating device with the through hole to align with the target branch bifurcation lumen by entirely displaying an inner wall contour of the inner wall within the lumen.

4. The medical catheter according to claim 3, wherein the sensor array can guide the distal hole of the coaxial rotating device with the through hole to align with the target bifurcation lumen by partially displaying the inner wall contour.

5. The medical catheter according to claim 1, wherein a detection device of the sensor array can be radially uniformly distributed at the distal end of the catheter.

6. The medical catheter according to claim 1, the detection device of the sensor array can be distributed in any fan-shaped area or radial arrangement around the catheter.

7. A medical catheter according to claim 3, wherein the sensor array is an ultrasonic transducer array, used to transmit and receive ultrasonic signals for detecting the inner wall contour of the lumen.

8. A medical catheter as claimed in claim 7, wherein an ultrasonic transducer array adjacent to a second ultrasonic transducer array transmits and receives signals one after another, and additional ultrasonic transducer arrays on both sides, or opposite sides from the adjacent ultrasonic transducer arrays of the catheter, transmit and receive signals at the same time to improve imaging efficiency.

9. A medical catheter as claimed in claim 7, wherein the ultrasonic transducer array resolves lumen bifurcations with a diameter of approximately 0.3mm to meet the desired requirement.

10. A medical catheter as claimed in claim 1, wherein the sensor array is an electromagnetic sensor or a photoelectric sensor, used to directly feedback position information of the distal end of the rotatable device relative to the catheter body.

11. The medical catheter according to claim 1, wherein the sensor array is an optical sensor.

12. A medical catheter as claimed in claim 1, wherein said fixation device is a balloon.

13. The medical catheter according to claim 1, wherein the fixing device is an elastic bracket.

14. A medical catheter according to claim 1, wherein the fixation device can be a nickel-titanium stent or a multi-link mechanism composed of hard materials with good biocompatibility, including, for example, titanium alloy or stainless steel.

15. A medical catheter as claimed in claim 1, wherein the fixation device has a driving end at the proximal end of the catheter, and the fixation device can be manually or automatically driven to switch states or deform to determine the distal end of the catheter and the lumen, and to stabilize the relative position of the catheter and lumen forming the channel.

16. The medical catheter according to claim 1, wherein a rotation mode of the coaxial rotation device can be manual.

17. The medical catheter according to claim 1, wherein a rotation mode of the coaxial rotation device can be driven by a computer-controlled motor.

18. A medical catheter as claimed in claim 1, wherein the coaxial rotation device can rotate independently relative to other parts of the catheter, and its rotation angle can be controlled.

19. A medical catheter as claimed in claim 1, wherein said coaxial rotation device, the catheter and the proximal end of the coaxial rotation device are connected with a mechanical structure, and the proximal end and distal end of the coaxial rotation device are connected with a flexible shaft that can rotate together or synchronously, such that the directional rotation of the coaxial rotating device and the distal hole on the coaxial rotating device, can be rotated so that the through hole is angularly aligned with the target branch bifurcation lumen to form the channel, and establishes a positional relationship connection between the through hole and the target branch bifurcation lumen forming the channel.

20. A medical catheter according to claim 1, wherein the relative angular relationship between the coaxial rotation device and the catheter can be provided by the proximal structure and/or the distal position sensor group.

21. A medical catheter as claimed in claim 1, wherein the relative angular relationship between the coaxial rotating device and the catheter can be transmitted via real time data to instruct an operator.

22. A medical catheter as claimed in claim 1, wherein the coaxial rotation device can guide a microcatheter or a guidewire to pass through a proximal inlet and out of the through hole through the distal hole.

23. A medical catheter as claimed in claim 1, wherein the distal end of the catheter includes a distal positioning device.

24. A medical catheter as claimed in claim 23, wherein the distal positioning device of the catheter is a radiopaque material.

25. A medical catheter as claimed in claim 23, wherein the distal positioning device of the catheter achieves the desired effect by spraying radiopaque material.

26. A medical catheter as claimed in claim 23, wherein the distal positioning device at the distal end of the catheter is a set of orthogonally placed coils.

27. A medical catheter as claimed in claim 23, wherein the distal positioning device of the catheter is a series of electrodes capable of transmitting electrical signals.
